# EUROPEAN PATENT APPLICATION

(11) **EP 2 463 874 A1**
(43) Date of publication of application: **13.06.2012**
(21) Application number: 11192049.2
(22) Date of filing: 06.12.2011
(51) Int. Cl.: H01H 9/02, A61G 13/10, A61B 19/00

(54) **Control console for a medical apparatus, the console being mountable on a rail**

(30) Priority: 13.12.2010 FR 1060406
(71) Applicant: Coactive Technologies, LLC, Newton, MA 04850 (US)
(72) Inventor: Nourry, Jean-François, 77580 Crecy La Chapelle (FR); Lecanu, Thomas, 93470 Coubron (FR)
(74) Representative: Kohn, Philippe

(57) **Abstract**

The invention relates to a control console (10) for an electronic medical apparatus, such as a radiography apparatus, the console (10) being designed to be mounted slidably on a transverse rail (16A), the console (10) comprising:
- a sealed housing (12) with a transverse groove (18) designed to be attached to the first rail (16A);
- a jaw (26), which is mounted movably on the housing (12) between a position in which the first rail (16A) is clamped between the jaw (26) and a rear face (19) of the housing (12), in order to immobilize the housing (12) with respect to the first rail (16A), and a position of release of the housing (12), in which position the housing (12) is able to be withdrawn from the first rail (16A);

characterized in that the jaw (26) is mounted movably on a deck (30), which is fixed on an outer face (33) of the sealed housing (12).

## Description

The invention relates to a control console for an electronic medical apparatus able to be mounted slidably on at least one rail.

The invention relates more particularly to a control console for an electronic medical apparatus, such as a radiography apparatus, the console being designed to be mounted slidably on at least a first transverse rail, the console comprising:
- a sealed housing with a transverse groove designed to be attached to the first rail;
- a jaw, which is mounted movably on the housing between a position in which the first rail is clamped between the jaw and a rear face of the housing, in order to immobilize the housing with respect to the first rail, and a position of release of the housing, in which position the housing is able to be withdrawn from the first rail.

Electronic medical apparatuses are generally controlled from electronic consoles. This is especially the case with certain radiography apparatuses using X-rays. The console is generally mounted on a horizontal rail, which can be fixed to a wall, to a hospital bed or even to a stretcher.

On account of its medical use, the control console is subject to very strict hygiene and safety standards. Therefore, the control electronics of the console are protected by being enclosed in a sealed housing. Moreover, from a hygiene point of view, a sealed housing of this kind is easily washable.

The console must also be able to be easily dismantled from the rails in order to use it on different rails.

The fixing means must also allow the console to slide on the rail in order to simplify the work of the medical personnel.

In the control consoles of the prior art, at least some elements of the fixing means are arranged inside the sealed housing.

This is unsatisfactory since maintenance work on the fixing means then requires dismantling the sealed housing. A maintenance procedure of this kind takes a great deal of time, since it is necessary to ensure that the housing is fully sealed after it has been re-assembled.

Moreover, during such a maintenance procedure, the electronic elements are exposed to the open air and risk causing malfunctions of the control console.

The invention proposes a control console of the type described above, characterized in that the jaw is mounted movably on a deck, which is fixed on an outer face of the sealed housing.

According to other features of the invention:
- the control device and the jaw are carried by the common deck in such a way as to form a module designed to be fixed against the outer face of the housing;
- the jaw is returned elastically to its clamping position;
- the movement of the jaw to its position of release is controlled by way of a drawer, which is mounted so as to slide horizontally on the deck;
- the drawer has locking means for locking the jaw in the clamping position thereof;
- the drawer is mounted so as to slide longitudinally under the deck;
- the console is also able to be mounted on a second rail, which is thicker than the first rail, the console being able to be immobilized on the second rail by said jaw; the groove being sufficiently wide to accommodate the second rail;
- the second rail is arranged in the continuation of the first rail; the second rail having an upper face arranged below the level of the upper face of the first rail; and the groove having a stepped profile, so as to permit the simultaneous bearing of the stepped arrangement on the first rail and the bearing of the base of the groove on the second rail when the console straddles the join between the two rails;
- the jaw has a corner, which allows the console to be immobilized in all directions with respect to the rail;
- the groove has a clearance, which allows the console to be tilted with respect to the rail when the jaw is in the position of release, in order to facilitate attachment/detachment of the console to/from the rail.

Other features and advantages will become clear on reading the following detailed description and by referring to the attached drawings, in which:
- Figure 1 is a perspective view showing a control console designed according to the teaching of the invention, the console being mounted in a position straddling two rails of different dimensions;
- Figure 2 is a perspective view seen from an opposite angle compared to Figure 1 and showing the console from Figure 1;
- Figure 3 is a longitudinal cross section along the sectional plane 3-3 in Figure 2;
- Figure 4 is an exploded perspective view showing the module comprising the means for fixing the console on the rails;
- Figure 5 is an exploded perspective view showing the module from Figure 4 assembled under the lower face of the console;
- Figure 6 is a longitudinal cross section along the sectional plane 6-6 in Figure 2.

In the description below, elements having identical, analogous or similar functions will be designated by the same reference numbers.

In the description below, the following orientations will be adopted without implying any limitation:
- longitudinal orientation "L", directed from the rear forwards;
- vertical orientation "V", directed from the bottom upwards; and
- transverse orientation "T", directed from left to right.

These orientations are indicated by the triple axis system "L,V,T" in the figures.

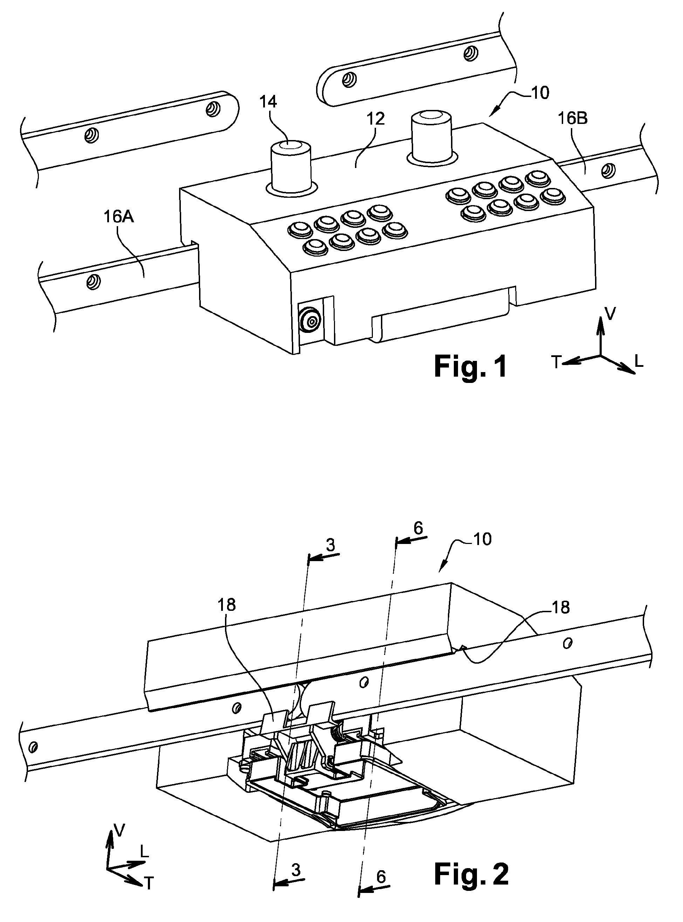

Figure 1 depicts a control console 10 for an electronic medical apparatus (not shown), for example a radiography apparatus using X-rays. This control console 10 comprises a sealed housing 12, which contains an electronic control unit (not shown).

In a known manner, a console 10 of this kind is fitted with various data input and/or output means, which are here formed by buttons and levers 14. The console 10 also comprises wired or wireless means (not shown) for communicating with the medical apparatus.

The console 10 is designed to be mounted on at least one rail 16 whose main orientation is a transverse orientation. In the example shown in the figures, the console 10 is able to be mounted on a first rail 16A and a second rail 16B, of which the cross sections are of different sizes. For this purpose, the console 10 comprises means for fixing on the rails 16A, 16B.

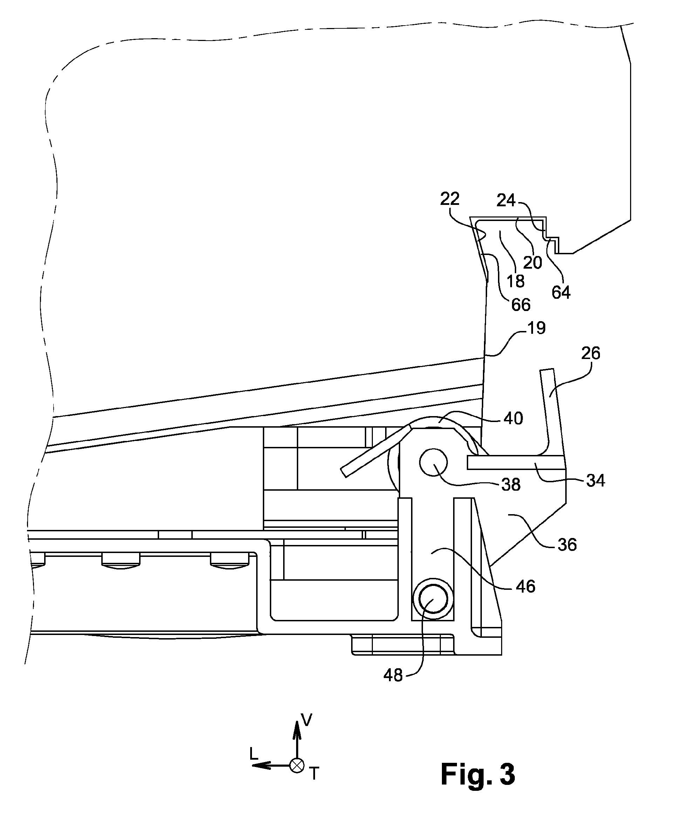

As is shown in Figures 2 and 3, the fixing means comprise at least one transverse groove 18, which is carried by a rear face 19 of the housing 12 and which is designed to be attached to the rails 16A, 16B. The groove 18 is open vertically towards the bottom and opens out transversely on both sides of the housing 12. The groove 18 is delimited vertically towards the top by a base 20 and longitudinally towards the front by a first transverse wall 22 and towards the rear by a second transverse wall 24. The groove 18 is thus designed to be attached to the rail 16A, 16B.

The groove 18 is here moulded integrally in the housing 12.

According to an alternative embodiment (not shown) of the invention, the groove is replaced by at least one hook, which is mounted on an outer face of the housing.

When the console 10 is attached to the rail 16A, 16B via its groove 18, the weight of the console 10 presses the rear face 19 of the housing 12 flat against the rail 16A, 16B by bearing on the upper edge of the rail 16A, 16B. The console 10 is thus attached in a stable manner.

To ensure that the console 10 attached in this way is fixed to the rail 16A, 16B, the console 10 also comprises at least one jaw 26, which is mounted movably on the housing 12 between:
- a position in which the rail 16A, 16B is clamped longitudinally between the jaw 26 and the rear face 19 of the housing 12, in order to immobilize the housing 12 with respect to the rail 16A, 16B, as is shown in Figure 3; and
- a position of release (not shown) of the housing 12, in which position the housing 12 is able to be withdrawn from the rail 16A, 16B.

It is possible for the jaw 26 to be actuated by a control device 28, which will be described in more detail below.

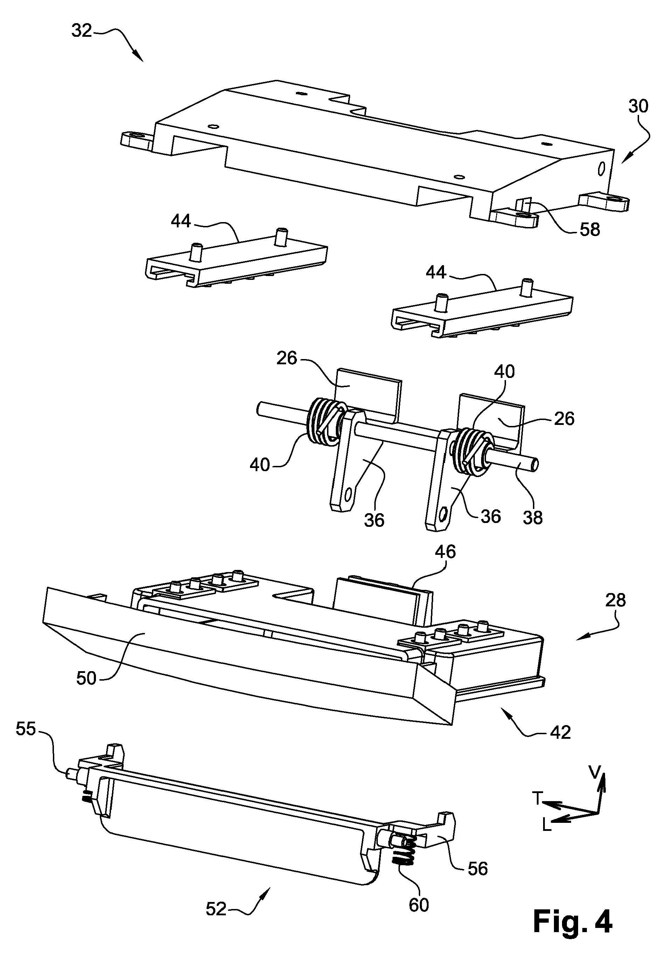

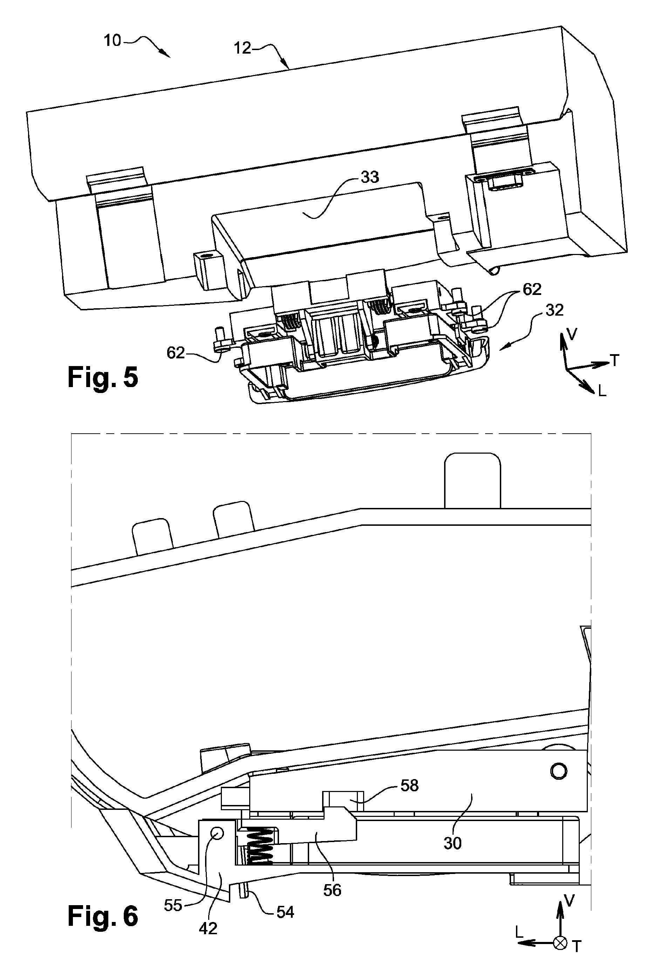

As is shown in Figure 4, the jaw 26 and the control device 28 are mounted on a common deck 30. The deck 30 is fixed under a lower face of the housing 12, as is illustrated in Figure 5.

Thus, the jaw 26 and the control device 28 form a module 32, which is fixed on an outer face 33 of the housing 12. This makes it possible in particular to easily service and repair the control device 28 without having to open the sealed housing 12.

The module 32 is shown in more detail in Figure 4. It comprises in particular the upper deck 30. The deck 30 is in the form of a horizontal panel.

The jaw 26 is formed by two transverse vertical tongues, which are carried by a common crosspiece 34. More particularly, each tongue forming the jaw 26 extends vertically upwards from a front end edge of the crosspiece 34.

The crosspiece 34 also carries two longitudinal vertical flanges 36, which extend vertically downwards from the lower face of the crosspiece 34.

The jaw 26 is mounted so as to pivot on the deck 30 about a transverse shaft 38, which is arranged substantially at the level of the crosspiece 34. The elastic return of the jaw 26 to its clamping position is effected by two torsion springs 40, which are engaged around the shaft 38, on either side of the flanges 36. Each torsion spring 40 has a first end, which bears under the crosspiece 34, and a second end, which bears under the deck 30, in such a way as to return the jaw 26 elastically to its clamping position.

The control device 28 comprises a drawer 42, which is mounted so as to slide longitudinally under the deck 30 by way of two lateral slides 44. For this purpose, the slides 44 are fixed under the deck 30, for example by means of screws. The drawer 42 thus slides between a rear clamping position, in which the jaw 26 occupies its clamping position, and a front release position, in which the jaw 26 occupies its position of release.

To permit the mechanical connection between the drawer 42 and the jaw 26, the drawer 42 has a transverse channel 46, which is delimited longitudinally by two transverse walls. The channel 46 opens vertically towards the top and also transversely on both sides. In the clamping position, the channel 46 is arranged vertically in line with the shaft 38 for pivoting the jaw 26. The channel 46 is then received transversely between the two flanges 36.

A transverse rod 48 is fixed between the lower ends of the two flanges 36. The rod 48 thus pivots along with the jaw 26 about the shaft 38. The rod 48 is thus received with longitudinal play in the channel 46. When the drawer 42 is pulled towards its front position of release, the rear wall of the channel 46 transforms the pulling force into a pivoting moment of the jaw 26 to its position of release. The rod 48 slides vertically upwards inside the channel 46 in order to permit the transformation of the longitudinal sliding movement of the drawer 42 into a pivoting movement of the jaw 26.

The drawer 42 additionally comprises a front handle 50 allowing an operator to pull the drawer 42 to its position of release.

In addition, in the example shown in the figures, the control device 28 comprises locking means for locking the drawer 42 in the clamping position thereof. The locking means are elastic engagement means of complementary shapes.

The locking means here comprise a lever 52, which is mounted so as to pivot under the drawer 42 about a transverse shaft 55. The lever 52 is shown in more detail in Figure 6. The lever 52 comprises a first, vertical actuation arm 54, which extends vertically downwards from the shaft 55. The first arm 54 forms a detent, which is arranged behind the handle 50 so as to be able to be pulled by an operator at the same time as the drawer 42.

The lever 52 also comprises two second, longitudinal arms 56, which extend longitudinally rearwards from the shaft 55. Each second arm 56 forms a hook. The hooks 56 are arranged transversely on either side of the drawer 42.

Each hook 56 is able to be engaged vertically upwards into an associated fixed notch 58 of the deck 30 when the drawer 42 is in its clamping position.

The lever 52 is thus mounted so as to pivot between an unlocked position, in which the hooks 56 are disengaged from their notches 58, and a locked position, in which the hooks 56 are engaged in their associated notch 58 in order to prohibit the sliding of the drawer 42 to its position of release with respect to the deck 30.

The lever 52 is returned elastically to its locked position. For this purpose, an associated spring 60 is interposed vertically between each hook 56 and a face of the drawer 42.

As is shown in Figure 5, the module 32 is fixed to the lower face 33 of the housing 12 by way of the deck 30. In the example shown in the figures, the lower face 33 of the housing 12 has a recess shape adapted to the module 32. Thus, the module 32 does not protrude below the housing 12.

The deck 30 here comprises four securing lugs 62, which are designed to receive screws for fastening to the housing 12.

According to an alternative embodiment (not shown) of the invention, the deck 30 is fixed on the housing 12 by elastic snap-fit means of complementary shapes.

Such a console 10 is thus able to be mounted on a single rail 16A, 16B by virtue of modular fixing means. The module 32 can be easily dismantled without the need to open the housing 12 of the console 10. This makes the maintenance procedures very much easier.

According to another aspect of the invention, the console 10 described above is also able to be mounted on two rails 16A, 16B of different dimensions. The two rails 16A, 16B are arranged in the continuation of one another. In the example shown in the figures, the second rail 16B has a longitudinal thickness greater than that of the first rail 16A. In addition, the second rail 16B has a vertical height less than that of the first rail 16A.

The groove 18 has a longitudinal width sufficient to accommodate the thicker second rail 16B.

In addition, the two rails 16A, 16B are fixed here in such a way that the upper edge of the first rail 16A is arranged above the level of the upper edge of the second rail 16B. The front faces of the two rails 16A, 16B are coplanar here.

To allow the console 10 to straddle the join between the two rails 16A, 16B, the rear wall 24 of the groove 18 has a stepped shape. Thus, the wall 24 has a horizontal shoulder face 64. Above the level of the shoulder face 64, the groove 18 has a longitudinal width substantially equal to that of the first rail 16A, whereas, below the level of the shoulder face 64, the groove 18 has a longitudinal width substantially equal to that of the second rail 16B.

Thus, the console 10 is able to bear vertically on the two rails 16A, 16B simultaneously. More particularly, when the console 10 straddles the join between the two rails 16A, 16B, the shoulder bears on the first rail 16A, and the base 20 of the groove 18 bears against the rear face of the second rail 16B. This allows the console 10 to maintain a horizontal attitude regardless of which rail 16A or 16B it is attached to. This also allows the console 10 to maintain a horizontal attitude when it is attached simultaneously to the two rails 16A, 16B by straddling the join.

When the console 10 thus straddles the join between the two rails 16A, 16B, the jaw 26 bears against the second, thicker rail 16B.

The groove 18 also comprises, in an upper part of its front face 22, a clearance 66 that allows the console 10 to be tilted with respect to the rail 16A, 16B when the jaw 26 is in the position of release, in order to make it easier for the console 10 to be attached to or detached from the rail 16A, 16B. The tilting of the console 10 makes it possible in particular to cause the jaw 26 to move to the position of release underneath the rails 16A, 16B.

According to an alternative embodiment (not shown) of the invention, the clearance is also applicable to a monorail console that has no stepped arrangement.

The stepped profile of the groove 18 thus makes it possible to obtain a very stable hold of the console 10.

According to an alternative embodiment (not shown) of the invention, the stepped shape of the rear wall is replaced by a slope. This allows the console to be adapted to several sizes of rails.

The dimensions of the steps are determined on the basis of the dimensions and arrangement of the rails 16A, 16B. The dimensions of the rails 16A, 16B are thus assumed to be known during the designing of the groove 18.

During the use of the console 10, an operator pulls the drawer 42 in order to make it slide towards its front position of release. This has the effect of causing the jaw 26 to tilt towards its position of release.

It is thus possible to attach the console 10 by way of its groove 18. By means of a tilting movement of the console 10 about a longitudinal axis permitted by the clearance 66, the jaw 26 in the position of release passes under the rails 16A, 16B.

The console 10 is then let go by the operator. The weight of the console 10 makes it possible to stabilize the console 10 in its position of use.

The jaw 26 and the drawer 42 are then returned elastically to their position of clamping the rail 16A, 16B between the jaw 26 and the rear face 19 of the console 10. The console 10 is thus fixed by clamping on the rail 16A, 16B.

By means of the crosspiece 34 that carries the jaw 26, it is possible to ensure that the console 10 cannot be moved vertically upwards with respect to the rail 16A, 16B. Indeed, if an attempt is made to lift the console 10, the crosspiece 34 comes into contact with the lower edge of the rail 16A, 16B in order to block the upward movement of the console 10.

When it is desired to move the console 10 on the rail 16A, 16B, it is possible to pull the drawer 42 towards its position of release. The jaw 26 is therefore no longer clamped against the rail 16A, 16B, and it is possible to slide the console 10 along the rail 16A, 16B without detaching the former.

By virtue of the stepped shape of the groove 18, it is possible to slide the console 10 from one rail 16A to the other rail 16B, situated in the continuation thereof, without detaching the console 10. For an operator, this transfer of the console 10 from one rail 16A, 16B to the other is done in the same way as sliding the console 10 on a single rail 16A, 16B.

Moreover, the arrangement of the module 32 outside the housing 12 greatly simplifies the maintenance procedures performed on the control device 28, without compromising the integrity of the control electronics contained in the housing 12.

## Claims

1. Control console (10) for an electronic medical apparatus, such as a radiography apparatus, the console (10) being designed to be mounted slidably on at least a first transverse rail (16A), the console (10) comprising:
- a sealed housing (12) with a transverse groove (18) designed to be attached to the first rail (16A);
- a jaw (26), which is mounted movably on the housing (12) between a position in which the first rail (16A) is clamped between the jaw (26) and a rear face (19) of the housing (12), in order to immobilize the housing (12) with respect to the first rail (16A), and a position of release of the housing (12), in which position the housing (12) is able to be withdrawn from the first rail (16A);
**characterized in that** the jaw (26) is mounted movably on a deck (30), which is fixed on an outer face (33) of the sealed housing (12).

2. Console (10) according to the preceding claim, **characterized in that** the jaw (26) is able to be actuated by a control device (28).

3. Console (10) according to the preceding claim, **characterized in that** the outer face (33) of the sealed housing (12) is configured as a recess in which the jaw (26) and its control device (28) are received.

4. Console (10) according to the preceding claim, **characterized in that** the control device (28) and the jaw (26) are carried by the common deck (30) in such a way as to form a module (32) designed to be fixed against the outer face (33) of the housing (12).

5. Console (10) according to any one of the preceding claims, **characterized in that** the jaw (26) is returned elastically to its clamping position.

6. Console (10) according to the preceding claim, **characterized in that** the movement of the jaw (26) to its position of release is controlled by way of a drawer (42), which is mounted so as to slide horizontally on the deck (30).

7. Console (10) according to the preceding claim, **characterized in that** the drawer (42) has locking means for locking the jaw (26) in the clamping position thereof.

8. Console (10) according to either of Claims 6 and 7, **characterized in that** the drawer (42) is mounted so as to slide longitudinally under the deck (30).

9. Console (10) according to any one of the preceding claims, **characterized in that** the console (10) is also able to be mounted on a second rail (16B), which is thicker than the first rail (16A), the console (10) being able to be immobilized on the second rail (16B) by said jaw (26);
and **in that** the groove (18) is sufficiently wide to accommodate the second rail (16B).

10. Console (10) according to the preceding claim, **characterized in that** the second rail (16B) is arranged in the continuation of the first rail (16A);
and **in that** the second rail (16B) has an upper face arranged below the level of the upper face of the first rail (16A);
and **in that** the groove (18) has a stepped profile, so as to permit the simultaneous bearing of the stepped arrangement on the first rail (16A) and the bearing of the base (20) of the groove (18) on the second rail (16B) when the console (10) straddles the join between the two rails (16A, 16B).

11. Console (10) according to any one of the preceding claims, **characterized in that** the jaw (26) has a corner, which allows the console (10) to be immobilized in all directions with respect to the rail (16A, 16B).

12. Console (10) according to the preceding claim, **characterized in that** the groove (18) has a clearance (66), which allows the console (10) to be tilted with respect to the rail (16A, 16B) when the jaw (26) is in the position of release, in order to facilitate attachment/detachment of the console (10) to/from the rail (16A, 16B).
